(19) Europäisches Patentamt
European Patent Office
Office européen des brevets

(11) Publication number: 0 284 725
A2

(12) # EUROPEAN PATENT APPLICATION

(21) Application number: 88101407.0

(51) Int. Cl.4: C07H 17/02 , A61K 31/70

(22) Date of filing: 01.02.88

(30) Priority: 03.02.87 US 10584
03.08.87 US 80731

(43) Date of publication of application:
05.10.88 Bulletin 88/40

(84) Designated Contracting States:
AT BE CH DE ES FR GB GR IT LI LU NL SE

(71) Applicant: QUEEN'S UNIVERSITY AT KINGSTON

Kingston Ontario K7L 3N6(CA)

(72) Inventor: Wolfe, Saul
R.R.No.1,
Kingston Ontario(CA)
Inventor: Bowers, Raymond J.
General Delivery
Bath Ontario(CA)

(74) Representative: MEISSNER, BOLTE & PARTNER
Widenmayerstrasse 48 Postfach 860624
D-8000 München 86(DE)

(54) **Indoxyl-beta-d-glucuronide and method for making.**

(57) Indoxyl-β- D -glucuronide, its stable salts and analogs thereof are described, together with a process for the production thereof in which a compound of the formula:

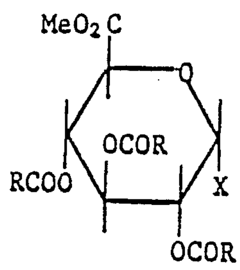

where X is halogen and R is lower alkyl is reacted with a compound of the formula:

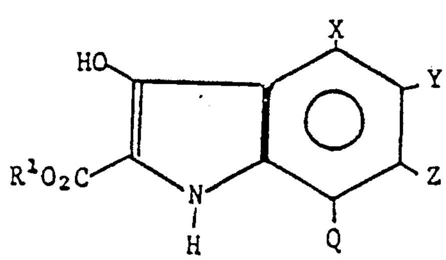

where $R^1$ is any common carboxyl-protecting group, X, Y, Z and Q are the same or different and

EP 0 284 725 A2

selected from hydrogen, halogen, trihalogenated alkene, trihalomethyl, nitro, saturated or unsaturated alkyl and an aromatic moiety in water and methylene chloride containing an alkali hydroxide in the presence of a phase transfer catalyst, with subsequent removal of the blocking groups and conversion to the desired stable salt.

# Indoxyl-β-D-Glucuronide and Method for Making

## Field of Invention

This invention relates to indoxyl-β- D -glucuronide and analogs thereof and to a method for the synthesis thereof.

## Cross Reference to Related Application

This application is a continuation-in-part of our earlier filed application for Letters Patent of the United States, Serial No. 010,584 filed 3 February 1987.

## Background of Invention

The fecal coliform test currently used to determine the presence of sewage in water is not specific for E.coli, which is the specific indicator for sewage. Bacteria found in food processing and pulp mill wastes also yield positive results in the fecal coliform test. It has been determined that E.coli has the ability to produce within itself the enzyme β- D -glucuronidase, whereas Enterobacter, Klebsiella and Citrobacter, commonly found in food processing and pulp waste, lack this ability. β- D -Glucuronidase has been previously identified in rat and dog tissues (see, for example "Laboratory Investigation" Vol.17, No.2, 217, 1967 "Histochemical β-Glucuronidase Distribution in Mammalian Tissue as Detected by 5-Bromo-4-Chloroindol-3-yl-β- D -glucopyuroniside", Pearson et al), by means of the synthetically prepared compound 5-bromo-4-chloroindol-3-y1-β- D -glucopyuronuride (A).

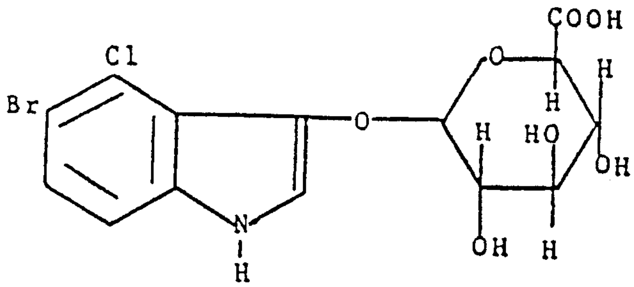

----------(A)

which is hydrolysed by the enzyme to form the insoluble, 5, 5-bromo-4,4′-chloroindigo as a bluish precipitate at enzymic sites. Previous attempts to make, by a variety of routes, indoxyl-β-glucuronides have, however, been unsuccessful, as the chemistry involved is somewhat complex and, for various reasons, the more obvious direct routes do not lead to the desired compounds (see, for example, "Tetrahedron" 1961, Vol.12, pp.236-239 "Substrates for the Histochemical localization of Some Gylcosidases", Anderson et al).

It has been postulated that indoxyl-β- D -glucuronide (I when X = COOH, Y = H) in the presence of β- D -glucuronidase would lead to the liberation of the aglycone 3-hydroxyindole, a substance which is converted spontaneously to the blue dye indigo

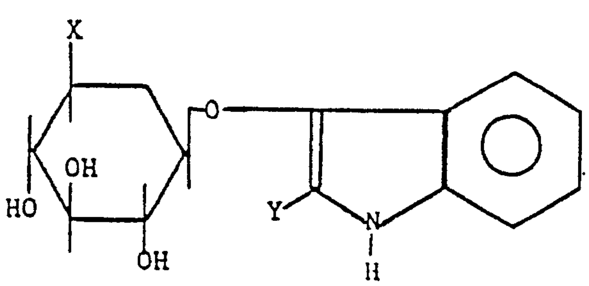

------------(I)

as it is known that the related compound indoxyl-β- D -glucoside (I where X = $CH_2OH$ and Y = H) will liberate indigo when exposed to a β-glucosidase enzyme as produced by Klebsiella, Citrobacter and Enterobacter. While the β-glucosidase system may be employed to distinguish between, on the one hand, E.coli and on the other hand Klebsiella, Citrobacter and Enterobacter, the system does not lend itself to specificity for a particular bacterium, the system is difficult to read and is subject to a high percentage of false positives. A system specific to E.coli is therefore to be preferred.

## Objects of Invention

An object of the present invention is to provide a process for the production of indoxyl-β- D -glucuronide, the stable salts and analogs thereof.

Another object of the present invention is to provide indoxyl-β- D -glucuronide, the stable salts and analogs thereof.

## Brief Statement of Invention

In satisfaction of the aforesaid objectives there is provided, by one aspect of the invention, compounds of the formula:

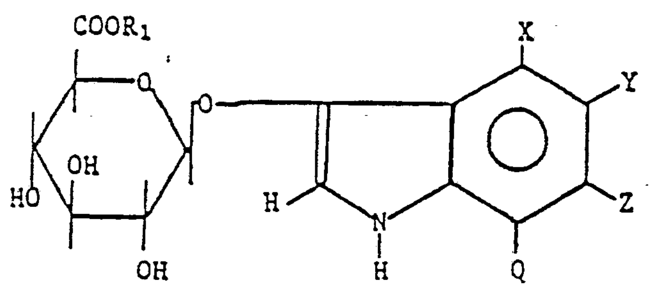

where X, Y, Z and Q are the same or different and selected from hydrogen, halogen, trihalogenated alkene, trihalomethyl, nitro, saturated or unsaturated alkyl, and an aromatic moiety provided that when X is chlorine, Y is not bromine; and $R_1$ is hydrogen, an alkali metal, lower alkyl ammonium or lower aryl ammonium.

By another aspect of the invention there is provided a process for the production of compounds having the formula:

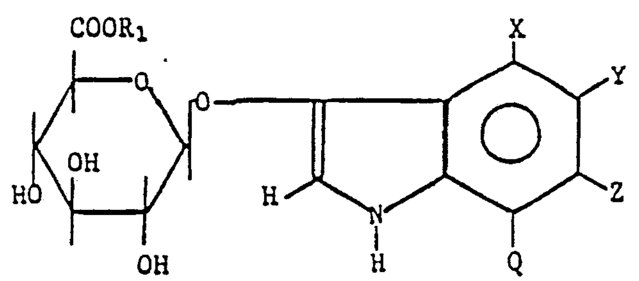

where X, Y, Z and Q are the same or different and selected from hydrogen, halogen, trihalogenated alkene, trihalomethyl, nitro, saturated or unsaturated alkyl, and an aromatic moiety; and $R_1$ is hydrogen, an alkali metal, lower alkyl ammonium or lower aryl ammonium,
comprising: reacting a compound of the formula:

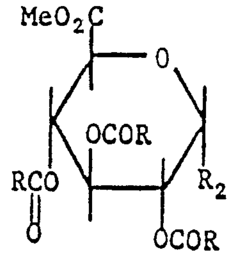

where $R_2$ is halogen and R is lower alkyl containing 1-3 carbon atoms with a compound of the formula:

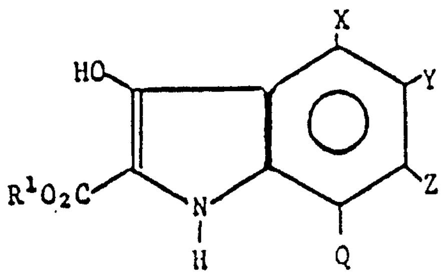

where $R_1$ is lower alkyl containing 1-5 carbon atoms or any of the commonly used carboxyl protecting groups, and X, Y, Z and Q are as above defined, in the presence of methylene chloride, an alkali metal hydroxide and a phase transfer catalyst, so as to produce a compound of the formula:

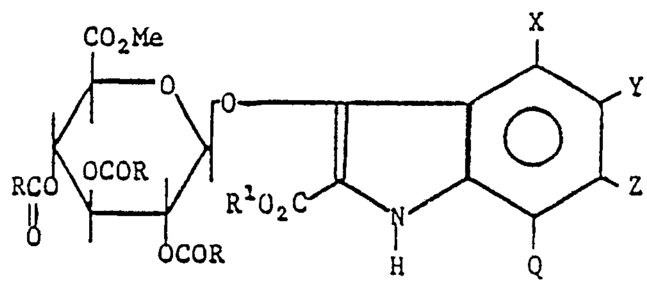

removing blocking groups therefrom so as to produce a compound of the formula:

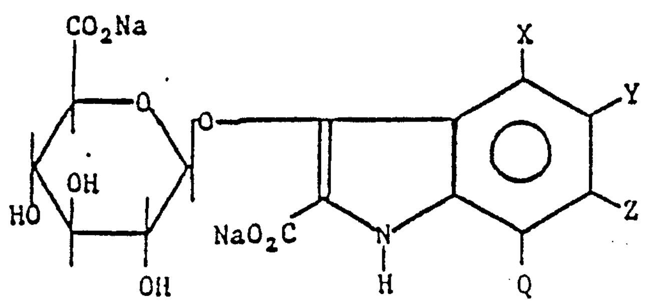

------------ (B)

removing sodium-containing groups therefrom to produce a compound of the formula:

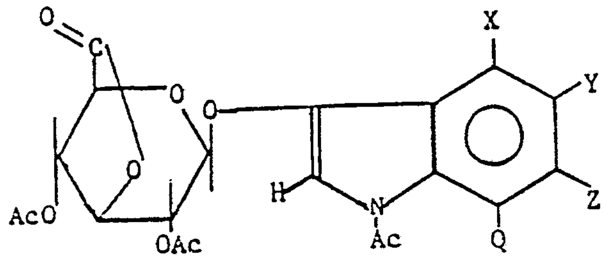

and reacting with an alkali metal, lower alkyl or aryl ammonium-containing base in methanol and water, removing methanol and freeze drying so as to produce a selected stable alkali salt product or, alternatively, heating the compound of the formula (B) in water at 200-250°C so as to produce the desired sodium salt directly.

Detailed Description of Invention

The present invention will be described in detail hereinafter with reference to the preferred embodiments for the production of indoxyl-β- D -glucuronide which has the formula:

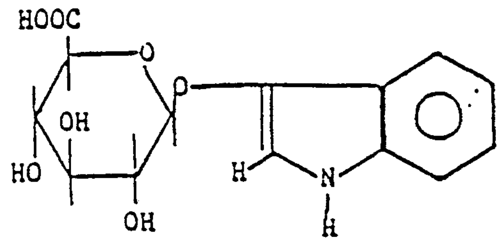

but it will be appreciated by those skilled in the art that the invention is equally applicable to analogs of indoxyl-β- D -glucuronide, having the general formula:

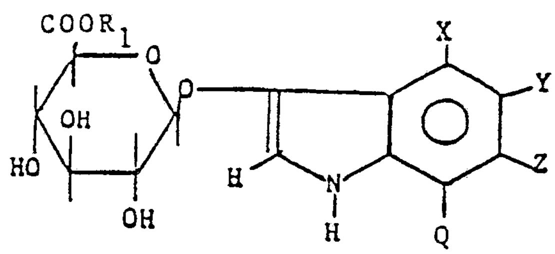

where X, Y, Z and Q are the same or different and selected from hydrogen, halogen, trihalogenated alkene, trihalo methyl, nitro, saturated or unsaturated alkyl or an aromatic moiety, provided that when X is chlorine, Y is not bromine; and $R_1$ is hydrogen, an alkali metal, lower alkyl ammonium or lower aryl ammonium.

Having determined that the synthesis of indoxyl-β- D -glucuronide from its glucoside precursor is not viable, as any attempt to oxidise $CH_2OH$ to COOH, regardless of conditions or oxidizing agent, invariably leads to instantaneous destruction of the indoxyl substrate, attention was directed to alternative readily available and relatively inexpensive starting materials.

Methyl [2,3,4-tri-O-acyl-β- D -glucopyranosyl halide] uronate

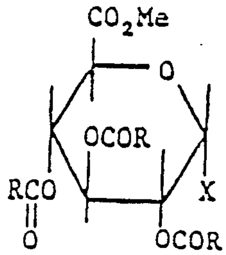

-----------(II)

where X is halogen and R is lower alkyl containing 1-3 carbon atoms was prepared from glucuronic acid or glucuronolactone by known techniques. Preferably in compound II, X = Br and R = $CH_3$.
2-carboxy-protected indoxyl,

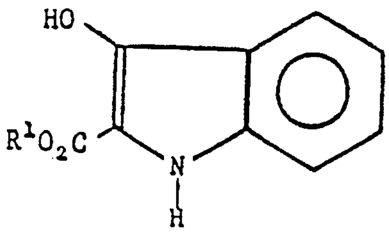

----------(III)

was prepared from methyl anthranilate,

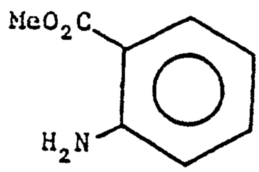

which may itself be prepared from anthranilic acid, by reaction with an ester of chloracetic acid ($ClCH_2CO_2R^1$) to give:

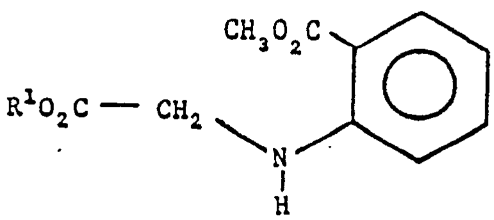

which is then exposed to sodium metal sodium hybride to give:

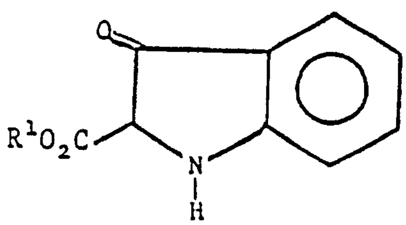

which is a tautomer of III.
Methyl [2,3,4-tri-O-acetyl-β- D -glucopyranosyl bromide] uronate (II, X = Br, R = $CH_3$)is then reacted with

2-carboxy-protected indoxyl, in water and methylene chloride containing an alkali metal hydroxide, preferably potassium hydroxide, and a phase transfer catalyst, such as tetra n-butyl ammonium hydrogen sulfate, to remove HBr with inversion of the configuration to produce methyl [(2-carboxy -protected indol-3-yl)-2,3,4-tri-O-acetyl-β- D glucopyranoside] uronate:

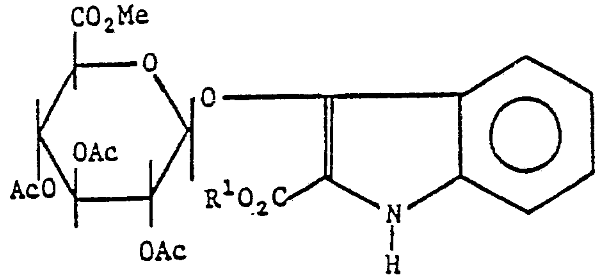

----------(IVa)

It will be appreciated by those skilled in the art that any phase transfer catalyst, known in the art, may be used (see: Dehmlow and Dehmlow "Phase Transfer Catalysis" Verlag Chemie 1980). Phase transfer catalysts include ammonium, phosphonium and sulfonium salts and chlorides, bromides or iodides may be substituted for hydrogen sulfate.

It is then necessary to remove all five blocking groups to produce disodium [(2-carboxyindol-3-yl)-β-D -glucopyranoside] uronate:

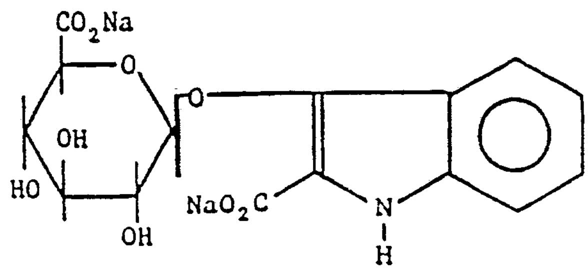

-----------(VI)

Removing the blocking groups may be effected either by reacting IVa with five molar equivalents of sodium hydroxide in water and methanol, or in stages by reacting IVa with barium methoxide to produce methyl [(2-carboxy-protected indol-3-yl)-β- D -glucopyrano-side] uronate:

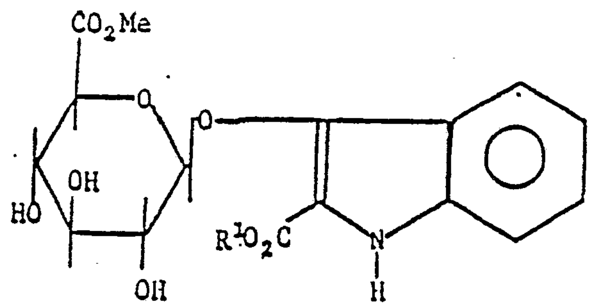

-----------(IV)

reacting IV with one equivalent of sodium hydroxide to produce sodium [(2-carboxy-protected indol-3-yl)-β-D -glucopyranoside] uronate:

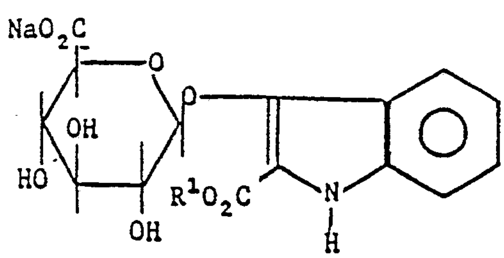

------------(V)

and reacting V with a further equivalent of sodium hydroxide to produce disodium [(2-carboxyindol-3-yl)-$\beta$-D -glucopyranoside] uronate:

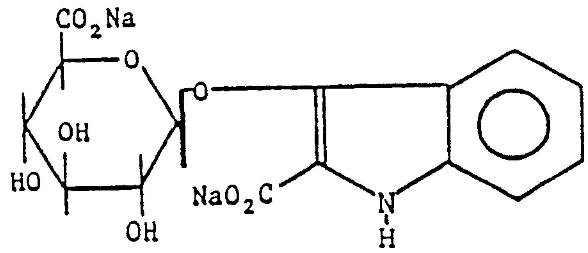

-----------(VI)

VI may then be reacted with acetic anhydride containing sodium acetate to remove $NaO_2C$ from the five membered ring and produce [(N-acetylindol-3-yl)-2,4-di-O-acetyl-$\beta$- D -glucopyranoside] uronate-3, 6-lactone:

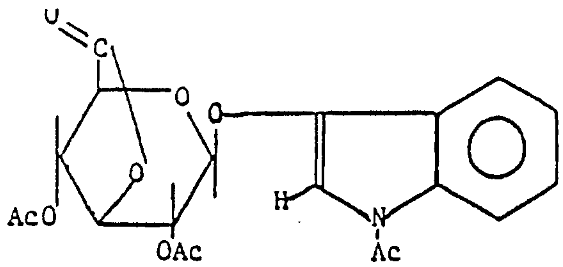

----------(VII)

in order to produce the salt of indoxyl-$\beta$- D -glucuronide or M-[(indol-3-yl)-$\beta$- D -glucopyranoside] uronate, VII may be reacted with one to four equivalents of any strong base MOH such as LiOH, NaOH or KOH in water of methanol, followed by evaporation to remove alcohol and freeze drying to give the salt of indoxyl-$\beta$-D-glucuronide:

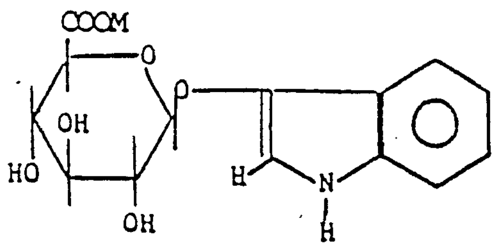

---------(VIII)

Those skilled in the art will appreciate that the term "strong base" as used herein includes any alkali metal hydroxide.

Alternatively, VI may be heated in water at 200-250°C to produce VIII directly.

Example 1

Preparation of Methyl [(2-carboxymethylindol-3-yl)-2,3,4-Tri-O-Acetyl-$\beta$- D -Glucopyranoside] Uronate

A mixture of methyl [2,3,4-tri-O-acetyl-$\beta$- D -glucopyranosyl bromide] uronate (4.58g, 11.5 mmole), 2-carboxymethyl-indoxyl (2.20g, 11.5 mmole), tetra n-butylammonium hydrogen sulfate (460 mg, 1.35 mmole) in water (25ml) and methylene chloride (25mL) was stirred vigorously at room temperature under nitrogen. To this was added 2.5 N aqueous potassium hydroxide (6.9 mL, 1.5 equiv) and the mixture was stirred for five and a half hours. After this time the methylene chloride was evaporated under reduced pressure and the residual black mixture was extracted with ethyl acetate (75 mL and 25 mL). The combined extracts were washed sequentially with water (100 mL), 1 N aqueous sodium hydroxide (2 x 50 mL), water (2 x 50 mL) and saturated brine (30mL). After drying over sodium sulfate the solvent was evaporated to leave a brown gum. The crude product was purified by column chromatography on silica gel, using toluene-

ethyl acetate 2:1 as eluant, to give a light tan foam ir(KBr): 1730(s,ester C=O), 1685(s,indole ester C=O, 1220(s,ester C-O)$cm^{-1}$; $^1$Hmr ($CDCl_3$)δ : 2.04(3H,s,OAc), 2.07(3H,s,OAc), 2.12 (3H,s,OAc), 3.72(3H,s,indole $CO_2CH_3$), 3.94(3H,s,uronate $CO_2CH_3$), 4.07(1H,d,J=9Hz,H5),5.27(1H,d,J=7Hz,H1),5.34-5.46-(3H,m,H2,H3,H4), 7.10 - 7.34 (3H,m,ArH), 7.82 (1H,d,J=8.5Hz,ArH), 8.63(1H,s,NH).

Example 2

Preparation of Methyl [(2-Carboxymethylindol-3-yl)-β- D -Glucopyranoside] Uronate

A solution of methyl [(2-carboxymethylindol-3-yl)-2,3, 4-tri-O-acetyl-β- D -glucopyranoside] uronate (l.0g, 1.97mmole) in dry methanol (150mL) was stirred at 5°C with 0.5M barium methoxide in methanol (1.5mL, 0.75mmole). After three hours the solution was warmed to room temperature and the reaction was quenched by bubbling carbon dioxide through the solution for thirty minutes. The solvent was evaporated and the residue was extracted with water (20mL), filtered, and freeze dried to leave a buff solid, m.p. 140-145°C(d); ir(KBr): 3360 (s,br,OH), 1730 (s,ester C=O), 1680 (s,indole ester C=O)$cm^{-1}$; $^1$Hmr($D_2O$) : 3.48-3.65(3H,m,H2,H3,H4), 3.66 (3H,s,indole $CO_2CH_3$), 3.76(3H,s,uronate $CO_2CH_3$), 3.86(1H,d,J=9.5Hz,H5), 4.83(1H,d,J=8Hz,H1), 6.99(1H,d,d, J=7.5 and 7.5Hz,ArH), 7.17-7.33(2H,m,ArH), 7.58(1H,d,J=8Hz,ArH).

Example 3

Preparation of Sodium [(2-carboxymethylindol-3-yl)-β- D -Glucopyranoside] Uronate

A solution of methyl [(2-carboxymethylindol-3-yl)-β - D -glucopyranoside] uronate (590mg, 1.55mmole) in methanol (15mL) was stirred at room temperature and treated, dropwise over one hour, with 0.1 N aqueous sodium hydroxide (15.5mL, 1.0 equiv). Water (2 x 2mL) was used to complete the addition of base and after a further two hours the solution was refluxed gently for forty minutes. Upon overnight storage at 4°c a fine white precipitate formed and was removed by filtration through Celite. The filtrate was freeze dried to leave a buff solid, ir(KBr): 3380 (br,OH), 1675 (s,br,indole $CO_2CH_3$), 1610 (s,$CO_2^-$)$cm^{-1}$; $^1$Hmr ($D_2O$)δ: 3.42-3.66(4H,m,H2,H3,H4,H5), 3.77 (3H,s,$CO_2CH_3$), 4.94(1H, d,J=8Hz,H1), 7.04 (1H,d,d,J=8.5 and 8.5Hz,ArH), 7.20-7.34 (2H,m, ArH), 7.71 (1H,d,J=8.5Hz,ArH); $^{13}$Cmr($D_2O$)δ: 52.71($CO_2CH_3$), 72.52, 73.82, 75.92, 77.35(uronate C2,C3,C4 and C5), 104.92(uronate C1), 113-18, 120.53, 120.94, 126.77 (indole CH), 114.41, 119.27, 134.61, 141.68 (indole C), 163.16 (indole $CO_2CH_3$), 176.14(uronate $CO_2$ ).

Example 4

Preparation of Disodium [(2-Carboxyindol-3-yl)-β- D -Glucopyranoside] Uronate

A solution of sodium [(2-carboxymethylindol-3-yl)-β- D -glucopyranoside] uronate (588mg, 1.51mmole) in water (1.5mL) and methanol (9.0mL) was treated with 1 N aqueous sodium hydroxide (1.5mL, 1.0 equiv) and refluxed gently for two hours. The slightly cloudy solution was cooled, filtered and evaporated to a small volume under reduced pressure. Dilution with methanol precipitated the product as an off white solid. A second crop was obtained from aqueous ethanol, m.p. 230°C(d); $^1$Hmr($D_2O$)δ: 3.38 (1H,d,d,J=9 and 9 Hz,uronate CH), 3.45-3.59(3H,m,uronate CH), 4.56 (1H,d,J=8Hz,uronate H1), 6.89 (1H,d,d,J=8 and 8Hz,ArH), 6.99 (1H,d,J=8HZ,ArH); $^{13}$Cmr($D_2O$)δ: 72.45= 73.63, 75.82, 77.46 (uronate C2,C3,C4, and C5), 106.43 (uronate C1), 113.04, 120.01, 120.82, 125.62 (indole CH), 120.53. 121.68, 133.26, 139.01 (indole C), 169.28 (indole $CO_2$ ), 176.36 (uronate $CO_2$ ).

Example 5

Preparation of [(N-Acetylindol-3-yl)-2,4-Di-O-Acetyl-β- D -Glycopyranoside] Uronate-3,6-Lactone

A slurry of disodium [(2-carboxyindol-3-yl)-β- D -glucopyranoside] uronate (402mg, 1.01mmole) and freshly fused sodium acetate (340mg, 4:1 equiv) in acetic anhydride (10.0mL) was stirred under nitrogen and placed in an oil bath at 100°C. The temperature of the oil bath was maintained at 115-120°C for thirty

minutes then raised to 140-145°C and held at this temperature for thirty minutes. The dark solution, which upon cooling to room temperature began to solidify, was quenched with iced water (200mL), stirred for two minutes, then extracted with ethyl acetate (2 x 25mL). The combined extracts were washed with saturated sodium bicarbonate (5 x 25mL) followed by saturated brine (20mL) then dried over sodium sulfate. Evaporation of the solvent gave a brown gum which was purified by column chromatography on silica gel using methylene chloride - ethyl acetate 19:1 as eluant. The product was obtained as an amber gum which crystallised readily from 98% ethanol. Recrystallisation from ethyl acetate - hexane gave buff microprisms, m.p. 180-182°C; 'Hmr($CDCl_3$)δ: 2.16(3H,s,OAc), 2.23(3H,s,OAc), 259(3H,s,NAc), 4.38 (1H,d,d,$J_{4,5}$ = 3.2Hz,$J_{3,4}$ = 0.6Hz,H5), 4.98(1H,d,d,$J_{4,5}$ = 3.2Hz,$J_{3,4}$ = 5.0Hz,H4), 5.24 (1H,m,$J_{3,4}$ = 5.0Hz,$J_{2,3}$ = 3.6Hz,$J_{5,3}$ = 0.6Hz,H3), 5.48 (1H,d,$J_{2,3}$ = 3.6Hz,H2), 5.54(1H,s,H1), 7.15(1H,s,ArH), 7.29(1H,d,d, J = 7.5 and 8.0Hz,ArH), 7.38(1H,d,d,J = 7.5 and 7.5Hz,ArH), 7.59(1H, d,J = 8.0Hz,ArH), 8.39 (1H,br,ArH).

Example 6

Preparation of [(N-Acetylindol-3-yl)-2,4-Di-O-Acetyl-β- D -Glucopyranoside] Uronate -3,6-Lactone From Methyl [(2-Carboxymethylindol-3-yl)-2,3,4-Tri-O-Acetyl-β- D -Glucopyranoside] Uronate

A solution of methyl[(2-carboxymethylindol-3-yl)-2,3, 4-tri-O-acetyl-β- D -glucopyranoside] uronate (102mg, 0.2mmole) in methanol (3.0mL) and water (2.0mL) was treated dropwise with 1 N aqueous sodium hydroxide (1.0mL, 5.0 equiv) and stirred at room temperature for 18 hours. After this time the methanol was evaporated under reduced pressure and the residual aqueous solution was freeze dried. The resultant buff solid was slurried with acetic anhydride (4.0mL) under nitrogen and placed in an oil bath at 100°C. The temperature of the oil bath was raised to 140°C and maintained at this temperature for thirty minutes. The reaction mixture was then cooled and quenched with iced water (100mL). The product was isolated and purified according to Example 5 and gave material identical in all aspects with that isolated in Example 5.

Example 7

Preparation of Indoxyl-β- D -Glucuronide Sodium Salt

A suspension of [(N-acetylindol-3-yl)-2,4-di-0-β- D -glucopyranoside] uronate-3,6-lactone (0.968g, 2.32mmoles) in methanol (100mL) and water (20mL) was cooled in an ice-bath, stirred, and treated dropwise during 15 min with 0.1 N sodium hydroxide (23.2mL, 1 equiv). The ice-bath was then removed and the mixture was allowed to warm to room temperature and stirred for a total of 12 h, during which time the solid dissolved. The solution was then refluxed for 2 h, cooled and evaporated to a partially crystalline glass. This was dissolved in methanol, and precipitated with ether to yield the sodium salt of indoxyl-β- D - glucuronide, m.p. 200-205°C. The compound has $R_f$ 0.48 (8:1:1 2-butanone:acetic acid:water) on silica gel, and is detected as a blue spot upon heating. 'Hmr($D_2O$)δ: 3.42-3.53(3H,m,H2,H3,H4), 3.62(1H, d,J = 9Hz,H5). 4.74 (1H,d,J = 7Hz,H1), 7.01 (1H,d,d,J = 8 and 8Hz,ArH), 7.07 (1H,s,Indole H2), 7-19 (1H,d,d,J = 8 and 8Hz,ArH), 7.31 (1H,d, J = 8Hz,ArH), 7.58 (1H,d,J = 8Hz,ArH).

Example 8

Conversion of Disodium [(2-Carboxyindol-3-yl)-β- D -Glucopyranoside] Uronate into Indoxyl-β- D - Glucuronide Sodium Salt by Heating in Water

The freeze-dried hydrolysis product of Example 6 (1.0g) was dissolved in water (3.0mL), and heated for 2 hours at 220-250°C in a sealed stainless steel apparatus. The reaction vessel was then cooled, opened, and the aqueous reaction mixture was extracted with ethyl acetate (10 x 5mL) and freeze-dried. The residue was triturated with methanol (4 x 10mL), and the methanol-soluable material was reevaporated. The residue was dissolved in water (20mL), and the solution was extracted successively with methyl isobutyl ketone (2 x 10mL) and n-butanol (3 x 10mL) and again freeze-dried to give the product, identified as indoxyl-β- D - glucuronide sodium salt on the basis of its chromatographic behavior, Hmr spectrum, and behavior in the assay described in Examples 10 and 11.

Example 9

Preparation of Indoxyl-β-D-Glucuronide Medium for use as a Reagent for Detecting E.coli

Enzyme activity was detected on the m-TEC medium of Dufour et al. (Dufour, A.P., E.R. Strickland and V.J. Cabelli. 1981. Membrane filter method for enumerating Escherichia coli. Appl. Envion. Microbiol. 41: 1152-1158) prepared with the following modifications: bromocresol purple, bromophenol red and lactose were omitted; the modified m-TEC medium contained (g/1): proteose peptone No. 3, 5.0g; yeast extract 3.0g; glycerol 10.0g; NaCl 7.5g; $K_2HPO_4$ 3.3g; $KH_2PO_4$ 1.0g; sodium lauryl sulfate 0.2g; sodium deoxycholate 0.10g and agar 15.0g. The medium was autoclaved for 15 minutes at 121°C. The following chemicals, purchased from the Sigma Chemical Company, St.Louis, Mo., were added separately to the modified m-TEC medium after autoclaving and cooling to 50°C - 60°C: para-nitrophenyl-β- D -glucuronide 400 mg/l; indoxyl-β- D -glucoside 250 mg/l. Indoxyl-β- D -glucuronide, prepared as described in Example 8, was added at 350-400 mg/l. The four agar media used in this study will be referred to as follows: modified m-TEC as m-TECB medium; the formulations of indoxyl-β- D -glucuronide as IGA, para-nitrophenyl-β- D -glucuronide as PNGA, and indoxyl-β- D -glucoside (indican) as IA media.

Example 10

Demonstration of Specificity of Indoxyl-β- D -Glucuronide for E.coli

In this experiment, four organisms were tested for β- D -glucuronidase and β- D -glucosidase activity: American Type Culture Collection (ATCC) strains Escherichia Coli 35218 and Klebsiella pneumonia 13883; environmental isolates that have been characterized in our laboratory, Escherichia coli 83-17350 and Klebsiella pneumonia PHL.

A series of m-TECB agar basal media microtiter plates were prepared, using the m-TECB base media described in Example 9, and indoxyl-β- D -glucuronide, p-nitrophenyl-β- D -glucuronide or indoxyl-β- D -glucoside was added to respective plates. Specific bacteria from controlled sources, described above, were applied to the plates and incubated at 45°C for 16 2 hours. The incubator plates were examined visually and the results are tabulated in Table I.

TABLE I

Reactions of Escherichia coli β-D-glucuronidase and Klebsiella pneumonia β-D-glucosidase activity on four media

| Bacteria | Enzyme reaction of bacteria on the following media: m-TECB | IGA | PNGA | IA |
|---|---|---|---|---|
| E. coli ATTC 35218 | - | + | + | - |
| E. coli 83-17350 | - | + | + | - |
| K. pneumonia ATCC 13883 | - | - | - | + |
| K. pneumonia PHL | - | - | - | + |

Note: + = positive reaction and - = negative reaction after growth at 44.5°C, 16 ±2 hr. Positive reactions on IGA, PNGA and IA were blue, yellow and blue colour development, respectively. Negative reactions on the four media were colourless.

The activity of E.coli β- D -glucuronidase and K. pneumonia β- D -glucosidase on m-TECB, IGA, PNGA or IA are shown in Table I. Environmental and ATCC strains of E.coli grown on IGA hydrolyse indoxyl-β- D -glucuronide and produce an indigo blue colour. Both E. coli strains liberate a diffuse yellow color on PNGA. E. coli exhibited no reactivity towards indican, a β- D -glucoside. In contrast to E. coli, K. pneumonia formed a blue colour from indican demonstrating β- D -glucosidase activity but was colourless on IGA and PNGA indicating the absence of the structural gene for β- D -glucuronidase. It can be seen from these results that indoxyl-β- D -glucuronide is readily and specifically hydrolysed by the glucuronidase activity of E. coli.

## Example 11

### Use of Indoxyl-β- D -Glucuronide for Enumerating E.coli in Water

A standard fecal coliform test for E.coli has been established by the Ontario Ministry of the Environment (Handbook of Analytical Methods for Environmental Samples, December 1983) and is known as the Membrane Filter Analysis Test. 250mL samples of well water and sewage known to contain E.coli were collected. The sewage samples were diluted 100 and 1,000 times with buffered water and then filtered under sterile conditions by vacuum filtration through 0.45mm pore size 47mm membrane filters. The filter membranes and a control membrane were then transferred to a petri dish containing m-TEC or IGA agar growth media as described in Example 9. The m-TEC and IGA agar plates were incubated at 44.5 ±0.5°C for 23 ± 1 hours. The fecal coliform colonies thus developed were counted using a stereoscopic microscope at 10X. The standard m-TEC samples showed yellow, yellow-brown and yellow-green colonies all of which were counted as fecal coliform colonies. The indoxyl samples showed blue colonies which were counted as E.coli colonies. The results are tabulated in Table II below:

TABLE II

| Source | Lab No. | Sample Volume (mL) | Number of E. coli and Fecal Coliform Colonies per Membrane Filter after Incubation on IGA or mTEC Medium<br>Filter<br>E.coli (IGA) | Fecal Coliform (M-TEC) |
|---|---|---|---|---|
| Well | 8968 | 1 | 18 | 23 |
| Well | 8969 | 1 | 15 | 12 |
| Sewage | 8954 | $10^{-3}$ | 14 | 13 |
| Sewage | 8955 | $10^{-2}$ | 127 | 162 |

Excellent recovery (78-100%) of E.coli was observed on the indoxyl-glucuronide medium (IGA) compared to the number of fecal coliforms found on m-TEC.

Example 12

Selectivity of Indoxyl-β-D-Glucuronide for E. Coli

Another laboratory tested IGA medium in two ways;
a) Twelve pure cultures were spotted onto a filter (preapplied to the IGA medium) with a wooden applicator stick. The plate was incubated at 35°C for 18 hours and the growth/colour of colonies assessed. The results are attached (Table III) and show that E. coli was the only organism producing a blue colony. b) Five samples from various sources were analyzed using IGA. Target (blue) and non-target colonies were picked, purified and identified.

Sixty-one target colonies were identified and only one did not contain E. coli (Table IV). Three of 28 non-target colonies contained E. coli of which one produced blue colonies when plated back onto IGA (Table V). The other two E. coli produced cream coloured colonies and thus are probably glucuronidase negative. The blue target colony from which E. coli was not isolated was a very pale blue and may actually have had E. coli present but due to overgrowth by the Klebsiella pneumoniae, identified from the colony, was not recovered when a purity streak was prepared.

Comparative counts were available from a Surface Water Lab for four of the samples (Table VI). When MF (Membrane Filter) was used in both labs there was a good correlation, however, the MPN (Most Probable Number) results were an order of magnitude lower than MF data on IGA. Possible causes of this difference may be more background inhibition of E.coli occuring in the MPN procedure or the procedure itself may be more inbibitory (MPN in EC-MUG @ 35°C for 2 hours and up to 72 hours at 44.5°C).

An Ontario Ministry of Health Laboratory tested the IGA medium using a number of E. coli isolates and a Klebsiella pneumoniae strain.

Appropriate dilutions of three strains of E. coli (a strong, a weak and non-glucuronidase strain) were seeded onto IGA by membrane filtration and incubated at 44.5°C for 18-20 hours. Both the strong and weak glucuronidase strains produced blue colonies while the negative strain (0157:H7) did not grow most likely due to temperature intolerance).

The Klebsiella pneumoniae (AT CC10031) produced a slightly yellow colony on IGA which was quite easily distinguished from the blue E. coli colonies.

A further test using ten environmental E. coli isolates from 8 different bathing beach samples was conducted. The pure cultures were spot inoculated onto a membrane filter placed on IGA medium and incubated 18-20 hours at 44.5°C. All E. coli produced blue colonies.

TABLE III

IGA Medium - Testing for Selectivity

1) Isolates (pure cultures) - applied to a membrane filter on IGA medium. Medium incubated 35°C, 18 hours.

| | Isolate | Reaction on IGA |
|---|---|---|
| 1) | E. coli (stock #3) | blue colony |
| 2) | K. pneumoniae (stock #7) | cream colony |
| 3) | S. typhimurium (stock #4) | cream colony |
| 4) | S. faecalis (stock #10) | no growth |
| 5) | S. aureus (stock #5) | cream colony |
| 6) | P. aeruginose (stock #21) | cream colony |
| 7) | Bacillus sp. (stock #22) | no growth |
| 8) | A. calcoaceticus (stock #64) | cream colony |
| 9) | Bacillus (stock #125) | no growth |
| 10) | A. hydrophila (stock #39) | cream colony |
| 11) | E. cloacae (0640-C) | cream colony |
| 12) | E. coli (weak Fl + on MUG) | blue halo surrounding cream colony |

TABLE IV

IGA MEDIUM - TARGET COLONY IDENTIFICATION

| Sample | Source | Blue Colonies Tested | % E. coli (or containing E. coli) |
|---|---|---|---|
| 1694 | Storm Sewer (Young's Creek) | 44 | 100% |
| HR | Humber River | 6 | 100% |
| 1890 | Centennial Park Sewer (St. Catharines Study) | 2 | 100% |
| 2793 | Domtar Construction final effluent (St. Catharines) | 9 | (8/9) = 88.9% |
| TOTAL | | 61 | (60/61) = 98.4% |

Total *E. coli* = 60 from target (blue) +3 from nontarget (cream) = 63

% of *E. coli* giving target reaction upon initial recovery = 60/63 = 95.2%

TABLE V

IGA MEDIUM - NONTARGET COLONY IDENTIFICATION

| Sample | Source | Background (cream) (Colonies tested) | % E. coli |
|---|---|---|---|
| 1694 | Storm Sewer (Young's Creek) | 8 | 1/8 = 12.5%. |
| HR | Humber River | 8 | 2/8 = 25% |
| 1890 | Centennial Park Sewer (St. Catharines) | 8 | 0 |
| 2793 | Domtar Final Effluent (St. Catharines) | 4 | 0 |
| TOTAL | | 28 | (*3/28 = 10.7%) |

* 1 of the 3 *E. coli* from an initial nontarget (cream) colony when plated back to IGAC gave a blue colony (1694-18B)

The other two (HR 7 and 8B) remain cream when replated to IGA.

TABLE VI

mTEC

| Sample | DIL | TAR CNT | BKD | mTEC MUG | IGA | CNT/100ml | |
|---|---|---|---|---|---|---|---|
| 1694 | 1 | 46 | 103 | 78 | 46000 | | 78000 RPTD |
| 1890 | 1 | | | 20 | | mTEC MUG | |
| | | | | | | | 20000 RPTD |
| | 2 | 2 | 102 | 9 | 20000 | | 90000 |
| 2793 | 1 | OBSC | - | | | | 9200 RPTD |
| | 1 | *62 | TNTC | | 62000 | MPN cECMUG | |
| 2791 | 1 | *53 | TNTC | | 5300 | | 240 RPTD |
| | 1 | 4 | 154 | | 4000 | | |
| HR | 1 | 6 | 268 | | | | |

RPTD = Result report
* Very heavy background growth
DIL = Dilution
TAR CNT = Target Count
BKD = Background
TNTC = Too numerous to count
MPN = Most probable number
OBSC = obscure
mTEC MUG = mTEC medium containing methyl-umbelliferone
B-D-Glucuronide
MPN cECMUG = MPN with methyl umbelliferone in EC medium.

**Claims**

1. Compounds of the formula:

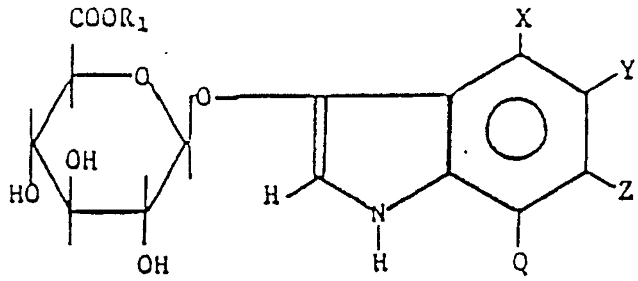

where X, Y, Z and Q are the same or different and selected from hydrogen, halogen, trihalogenated alkene, trihalo methyl, nitro, saturated or unsaturated alkyl, and an aromatic moiety provided that when X is chlorine Y is not bromine; and R is hydrogen, an alkali metal, lower alkyl ammonium or lower aryl ammonium.

2. A compound as claimed in Claim 1 wherein X, Y, Z and Q are hydrogen.

3. A compound as claimed in claim 2 wherein $R_1$ is an alkali metal, lower alkyl ammonium or lower arylammonium.

4. A compound as claimed in Claim 3 wherein $R_1$ is sodium.

5. A compound as claimed in Claim 3 wherein $R_1$ is cyclohexylammonium.

6. A process for the production of compounds having the the formula:

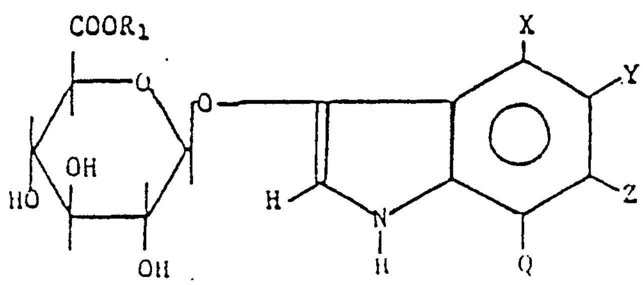

where X, Y, Z and Q are the same or different and selected from hydrogen, halogen, trihalogenated alkene, trihalo methyl, nitro, saturated or unsaturated alkyl, and an aromatic moiety; and $R_1$ is hydrogen, an alkali metal, lower alkyl ammonium or lower aryl ammonium,
comprising: reacting a compound of the formula:

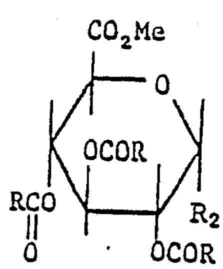

where $R_2$ is halogen and R is lower alkyl containing 1-3 carbon atoms
with a compound of the formula:

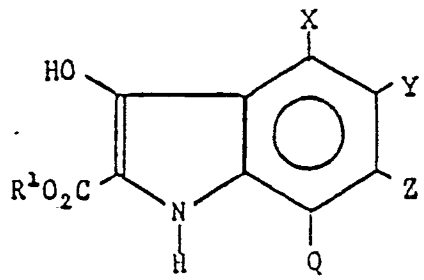

where $R_1$ is any common carboxyl-protecting group and X, Y, Z and Q are as above defined, in the presence of methylene chloride, an alkali metal hydroxide and a phase transfer catalyst, so as to produce a compound of the formula:

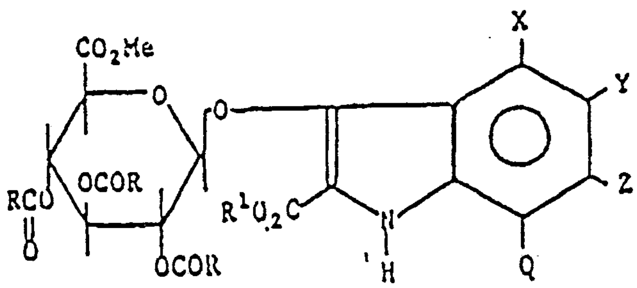

removing blocking groups therefrom so as to produce a compound of the formula:

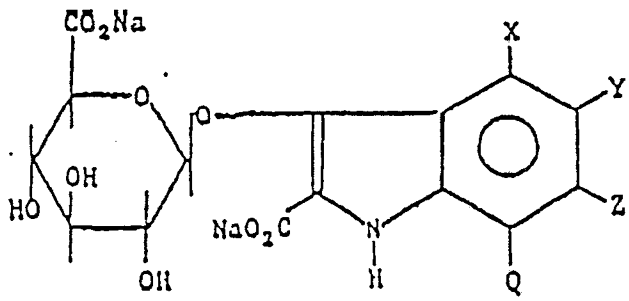

removing sodium-containing groups therefrom to produce a compound of the formula:

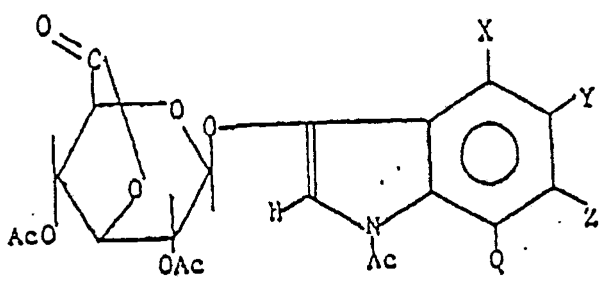

and reacting with an alkali metal, lower alkyl or aryl ammonium-containing base in methanol and water, removing methanol and freeze drying so as to produce a selected stable alkali salt product.

7. A process as claimed in Claim 6 wherein $R_2$ is bromine; X, Y, Z and Q are hydrogen; $R_1$ is sodium; and R and $R^1$ are methyl.

8. A process as claimed in Claim 7 wherein a compound of the formula:

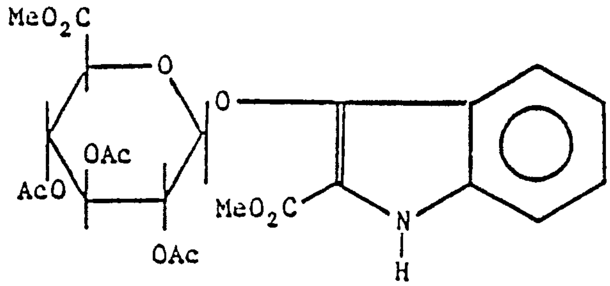

is reacted with five equivalents of sodium hydroxide in water and methanol so as to produce a compound of the formula:

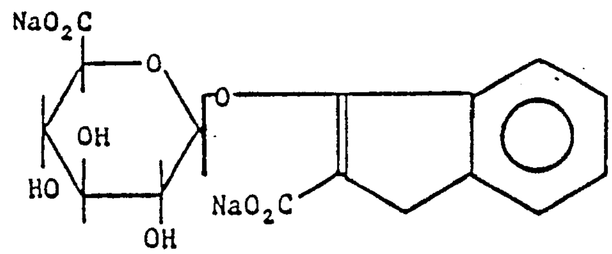

9. A process as claimed in Claim 7 wherein a compound of the formula:

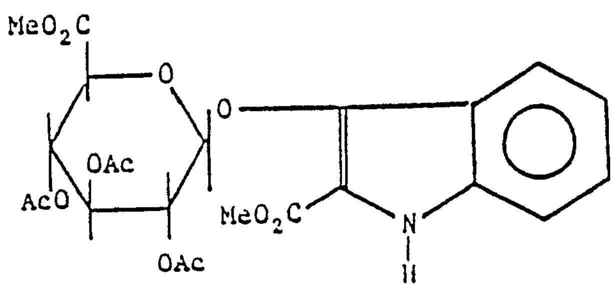

is reacted with barium methoxide to produce:

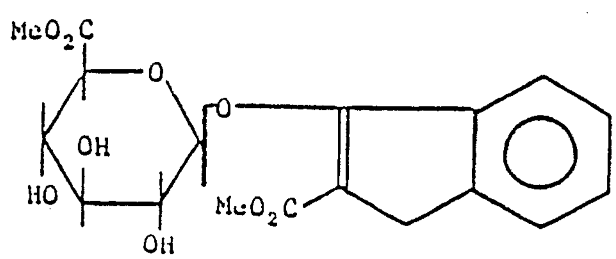

which is then reacted with NaOH to produce:

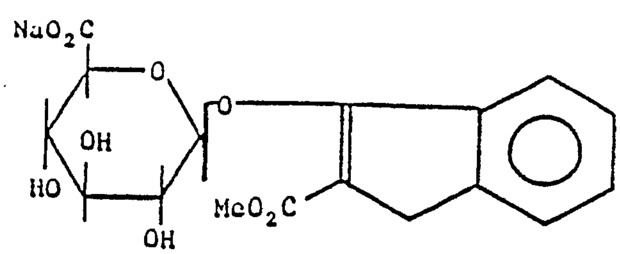

which is then reacted with NaOH to produce:

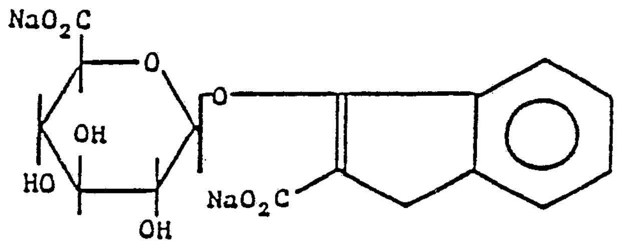

10. A process for the production of compounds having the formula:

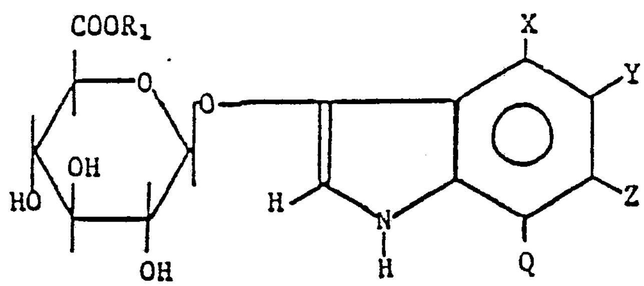

where X, Y, Z and Q are the same or different and selected from hydrogen, halogen, trihalogenated alkene, trihalo methyl, nitro, saturated or unsaturated alkyl, and an aromatic moiety; and $R_1$ is hydrogen, an alkali metal, lower alkyl ammonium or lower aryl ammonium,
comprising: reacting a compound of the formula:

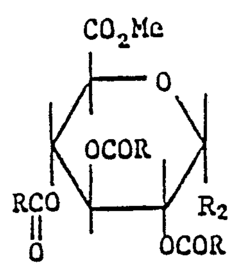

where $R_2$ is halogen and R is lower alkyl containing 1-3 carbon atoms
with a compound of the formula:

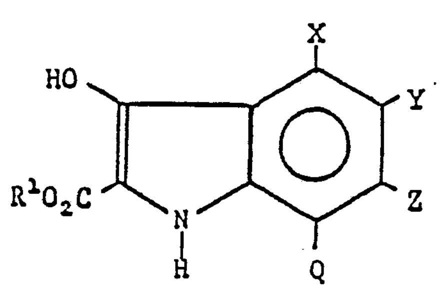

where $R^1$ is any common carboxyl-protecting group and X, Y, Z and Q are as above defined,
in the presence of methylene chloride, an alkali metal hydroxide and a phase transfer catalyst, so as to produce a compound of the formula:

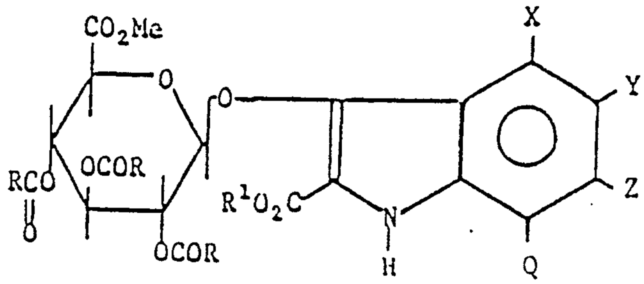

removing blocking groups therefrom so as to produce a compound of the formula:

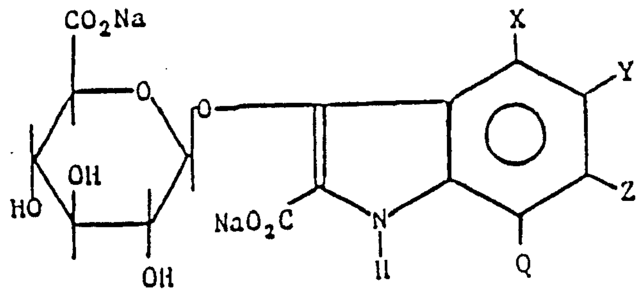

which is then converted into a selected, stable alkali salt product by heating in water.

11. A process as claimed in Claim 10 wherein $R_2$ is bromine; X, Y, Z and Q are hydrogen; $R_1$ is sodium; and R and $R^1$ are methyl.